# EUROPEAN PATENT SPECIFICATION

(11) **EP 2 194 835 B1**
(45) Date of publication and mention of the grant of the patent: **12.02.2014**
(21) Application number: 08834573.1
(22) Date of filing: 26.09.2008
(51) Int. Cl.: A61B 1/00, A61B 1/04, G02B 23/24, H04N 5/225

(54) **IMAGE PICKUP APPARATUS AND ENDOSCOPE HAVING THE SAME**
BILDAUFNAHMEGERÄT UND ENDOSKOP MIT EINEM SOLCHEN
APPAREIL DE PRISE D'IMAGES ET ENDOSCOPE LE COMPRENANT

(30) Priority: 28.09.2007 JP 2007256700
(43) Date of publication of application: 16.06.2010
(73) Proprietor: FUJIFILM Corporation, Tokyo 106-8620 (JP)
(72) Inventor: KIDO, Takashi, Kurokawa-gun Miyagi 981-3496 (JP); SUZUKI, Hisashi, Kurokawa-gun Miyagi 981-3496 (JP)
(74) Representative: Klunker . Schmitt-Nilson . Hirsch
(86) International application number: PCT/JP2008/067985
(87) International publication number: WO 2009/041724

(56) References cited:
- JP-A- 4 218 136
- JP-A- 10 014 868
- JP-A- 11 271 646
- JP-A- 2004 207 461
- JP-A- 2006 186 483
- JP-A- 2006 280 940
- US-A- 5 220 198
- US-A1- 2004 130 640
- US-A1- 2004 167 378
- US-A1- 2006 264 083

## Description

### Technical Field

The present invention relates to an image pickup apparatus according to the preamble of claim 1 and an endoscope having the same.

### Background Art

Biomedical and electronic endoscope devices which are to be inserted into body cavity for internal observation have been developed. Generally, an electronic endoscope has a lens and a solid state image pickup element (CCD) provided at the tip portion thereof. An image observed by the endoscope is focused on the solid state image pickup element via the lens for photoelectric conversion. The observed image after photoelectric conversion is represented by an electrical signal, which is appropriately processed by a processor to be output to a monitor TV, where the observed image is displayed.

For various reasons, there is a need for miniaturization of the image pickup apparatus with a solid state image pickup element which is provided at a tip portion of an electronic endoscope.

For miniaturization of an image pickup apparatus, Patent Document 1 discloses a structure in which one circuit board parallel to a solid state image pickup element is connected to the other circuit board which is disposed in a perpendicular direction to the circuit board to form a substantially L-shaped so that components that cannot be mounted on the parallel circuit board are mounted to the perpendicular circuit board for miniaturization. Patent Document 2 discloses a structure of an image pickup apparatus in which a circuit board is extended from a solid state image pickup element and bent to form a U-shaped configuration, so that a cable is sandwiched within the bent board portion for miniaturization of the image pickup apparatus. Patent Document 3 discloses a structure of an image pickup apparatus in which a circuit board is bent in a predetermined shape and the circuit board has two surfaces, to which external wiring is connected and components are mounted using through holes formed in the circuit board for miniaturization of the image pickup apparatus.
[Patent Document 1] Japanese Patent Application Laid-Open No. 2007-7429
[Patent Document 2] Japanese Patent Application Laid-Open No. 10-14868
[Patent Document 3] Japanese Patent Application Laid-Open No. 11-271646

In accordance with the preamble of claim 1, US 5 220 198 A discloses an image pickup apparatus according to the preamble of claim 1, in which according to one embodiment a bendable circuit board is attached to the back surface of the solid state image pickup element, wherein the bendable circuit board forms a L-shaped cross section. The surface of the circuit board which is facing away from the solid state image pickup element carries an electronic device.

US 2006/0264083 A1 discloses a capsule-type endoscope in which a plurality of rigid circuit boards is interconnected by small stripes of flexible circuit boards. The rigid boards can be placed one on another by alternately folding the flexible boards in opposite directions.

### Disclosure of the Invention

### Problems to be solved by the Invention

However, the image pickup apparatus described in Patent Document 1 requires extra components to connect between the plurality of circuit boards, and the step for mounting the circuit boards is complicated due to the different directions for each connection. In the image pickup apparatus described in Patent Document 2, the circuit board is extended from a back surface of a solid state image pickup element in a direction perpendicular to the surface, which increases the length of the circuit board. In the image pickup apparatus described in Patent Document 3, electrical components are mounted to both of the surfaces of a circuit board, which complicates the step for mounting the components.

The present invention was made in view of the above situation, and one object of the present invention is to provide an image pickup apparatus which is miniaturized and easily assembled and can be mounted to a tip portion of an endoscope device.

### Means for Solving the Problems

In order to achieve the above object, an image pickup apparatus according to an aspect of the present invention comprises the features of claim 1.

In the image pickup apparatus according to the above aspect of the present invention, the image pickup apparatus further comprises at least two of the electronic components, and the circuit board is bent to cause the electronic components to be opposed to each other.

Since the circuit board is bent to cause the electronic components to be opposed to each other, the mounting density of the components is increased. The electronic components which could not be received in the projection area of the solid state image pickup element are mounted in parallel to the solid state image pickup element, which prevents the overall length of the image pickup apparatus from being increased.

In the image pickup apparatus according to the above aspect of the present invention, the opposed electronic components are sealed by resin.

Since the circuit board is bent so that the electronic components are disposed opposed to each other, the electronic components are received in the space which is formed by the bending of the circuit board. The circuit board serves as a kind of mold, thereby a filling of the space with resin allows the electronic components to be fixed together.

According to the aspect of the present invention, a back surface of a solid state image pickup element and a circuit board are connected to each other, thereby the heat generated at the solid state image pickup element can be radiated through the circuit board. The bendable circuit board is bent within the tip portion of an endoscope device, which enables the downsizing of the image pickup apparatus in its radial direction. The electronic components are mounted to one surface of a circuit board, which facilitates an assembly of the apparatus, and also the surface of the circuit board to which electronic components are mounted is disposed substantially in parallel to the back surface of the solid state image pickup element, which decreases the length of the image pickup apparatus. This enables an effective use of the space on the back surface side of the solid state image pickup element. In spite of the flexibility of the base of the circuit board, the rigidity of the solid state image pickup element is supposed to improve the reliability in mounting electronic components.

In the image pickup apparatus according to the above aspect of the present invention, it is preferable that the signal cable is connected to the circuit board at one surface thereof to which electronic components are mounted.

Since a signal cable and electronic components are disposed on the same surface of the circuit board, the signal cable and the electronic components can be easily connected to each other and mounted to the circuit board in an assembly step.

In the image pickup apparatus according to the above aspect of the present invention, it is preferable that the other surface of the circuit board to which the electronic components are not mounted is connected to the back surface of the solid state image pickup element.

Since the other surface of the circuit board is connected to the back surface of the solid state image pickup element, the strength of the fixed part of the circuit board is increased, which improves the reliability in mounting electrical components. In addition, the circuit board is partly fixed to the solid state image pickup element, which facilitates the assembling of a tip portion of an endoscope device having the element.

In the image pickup apparatus according to the above aspect of the present invention, it is preferable that the circuit board has a conductor layer on the other surface, and the conductor layer and the back surface of the solid state image pickup element are thermally connected to each other.

Since a conductor layer formed on the other surface and the back surface of the solid state image pickup element are thermally connected to each other, the heat generated at the solid state image pickup element can be efficiently radiated from the circuit board.

In the image pickup apparatus according to the above aspect of the present invention, it is preferable that the conductor layer is electrically connected to the back surface of the solid state image pickup element.

Since the conductor layer and the back surface of the solid state image pickup element are electrically connected to each other, the conductor layer is able to function as a ground pattern.

In the image pickup apparatus according to the above aspect of the present invention, it is preferable that the conductor layer is connected to a shield of the cable.

Since the conductor layer is connected to the shield of the cable, any electromagnetic waves from the outside can be blocked and any emission of input/output signals to the outside can be restrained, so as to maintain the quality of the image signals.

In the image pickup apparatus according to the above aspect of the present invention, it is preferable that the image pickup apparatus further comprises a cylinder which partly covers the solid state image pickup element, the circuit board, the electronic component, and the cable, and the cylinder and the conductor layer are connected to each other.

Since the cylinder and the conductor layer are connected to each other, any electromagnetic waves from the outside can be blocked and any emission of input/output signals to the outside can be restrained, so as to maintain the quality of the image signals.

In the image pickup apparatus according to the above aspect of the present invention, it is preferable that the image pickup apparatus further comprises a lens barrel which is provided on the main surface of the solid state image pickup element for holding the optical system therein, and the lens barrel and the conductor are connected to each other.

The heat generated at the solid state image pickup element is radiated to the lens barrel, which prevents any dew condensation from being formed on the surface of a cover glass. A temperature rise due to the heated solid state image pickup element causes a thermal gradient (temperature difference) to be established between the cover glass and the solid state image pickup element, which produces dew condensation on the surface of the cover glass. When the heat generated at the solid state image pickup element is radiated to the lens barrel, the cover glass is heated and any thermal gradient (temperature difference) is hardly established between the cover glass and the solid state image pickup element.

In the image pickup apparatus according to the above aspect of the present invention, it is preferable that the opposed electronic component are disposed in a staggered arrangement.

The electronic components are disposed opposed to each other and also in a staggered arrangement, which enables an effective use of a space and further increases the mounting density.

In the image pickup apparatus according to the above aspect of the present invention, it is preferable that the connection portion is a through-hole interconnection formed in the solid state image pickup element.

Since a through-hole interconnection is used to electrically connect the solid state image pickup element to the circuit board, which eliminates any connection formed on the peripheral portions of the solid state image pickup element. As a result, the image pickup apparatus can be downsized in the radial direction thereof. Also, the use of through-hole interconnection enables an efficient transference of heat generated at the solid state image pickup element to the circuit board.

In the image pickup apparatus according to the above aspect of the present invention, it is preferable that the image pickup apparatus further comprises an underfill agent which is filled between the solid state image pickup element and the circuit board.

The filling of an underfill agent prevents any connection problems which are caused by stress due to a difference between the heat expansions of the solid state image pickup element and the circuit board, impact shock, external stress by bending, and the like.

An endoscope of the present invention is characterized by comprising the above described image pickup apparatus.

### Advantages of the Invention

According to the present invention, an image pickup apparatus which is miniaturized and easily assembled to be mounted to a tip portion of an endoscope is provided.

### Brief Description of the Drawings

Figure 1 is a view showing the entire structure of an endoscope system;
Figure 2 is a perspective view showing the tip portion of the endoscope;
Figure 3 is a cross sectional view showing a first embodiment of an image pickup apparatus according to the present invention;
Figure 4 is a cross sectional view showing a second embodiment of an image pickup apparatus according to the present invention;
Figure 5 is a cross sectional view showing a third embodiment of an image pickup apparatus according to the present invention; and
Figures 6A to 6E are views illustrating the assembly procedure of an image pickup apparatus according to the present invention.

### Description of Symbols

100 ... image pickup apparatus, 108 ... cover glass, 110 ... solid state image pickup element, 112 ... light receiving section, 114 ... electrode, 116 ... flexible circuit board, 122, 124 ... electronic components, 130 ... signal cable, 132 ... core wire, 138 ... wire conductor, 140 ... conductor pattern

### Best Mode for Carrying Out the Invention

Now, preferred embodiments of the present invention will be explained below in accordance with the appended drawings. The present invention will be explained by way of the preferred embodiments, but many approaches may be used to add changes to the present invention without departing from the scope of the present invention, and also many embodiments other than the following embodiments can be attained. Therefore, any changes within the scope of the present invention will be included in the scope defined by the claims.

### [Entire System of Endoscope Device]

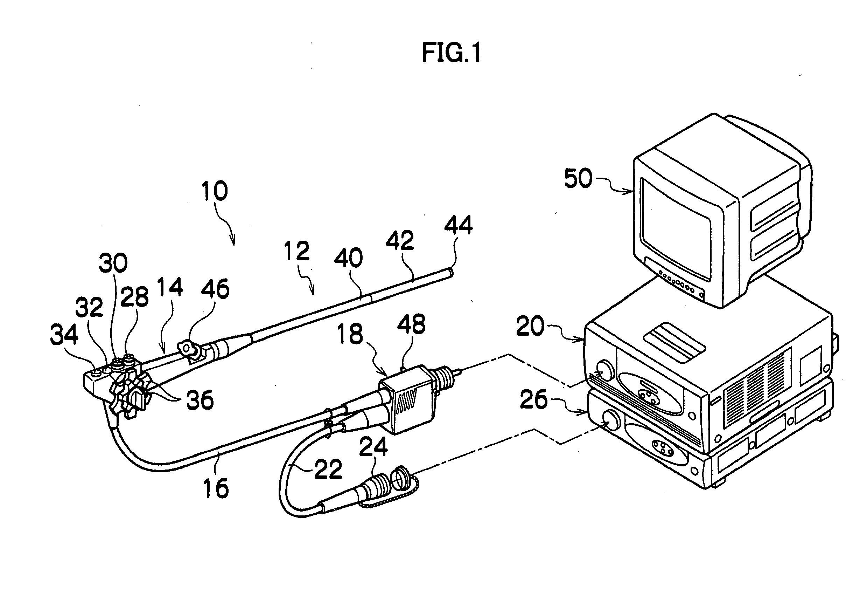

Figure 1 shows the entire structure of an endoscope device system in which an image pickup apparatus of the present invention is used.

As shown in Figure 1, the endoscope device includes an endoscope 10. The endoscope 10 includes a proximal operation unit 14 and an insertion unit 12 which is linked to the proximal operation unit 14 and is to be inserted into a body cavity. The proximal operation unit 14 is connected with a universal cable 16 that has a tip portion provided with an LG connector 18. The LG connector 18 is removably coupled a light source device 20. Through the LG connector 18, an illumination light is transmitted to an illumination system (not shown). Also, the LG connector 18 is connected with an electrical connector 24 via a cable 22. The electrical connector 24 is removably coupled to a processor 26.

The proximal operation unit 14 is provided with an air-supply/water-supply button 28, a suction button 30, a shutter button 32, a function switch button 34, and a pair of angle knobs 36.

The insertion unit 12 is configured with a soft portion 40, a curving portion 42, and a tip portion 44, in order from the proximal operation unit 14 side. The curving portion 42 is remotely controlled to curve by a rotation of the angle knobs 36 of the proximal operation unit 14. This allows the tip portion 44 to be directed toward a desired direction.

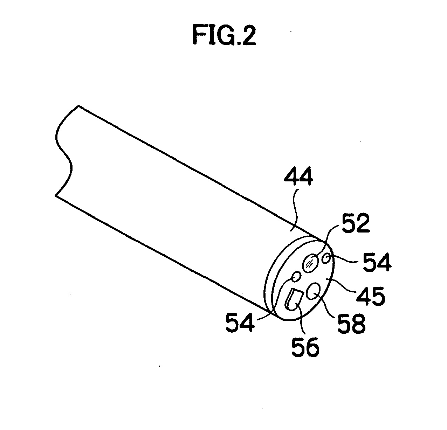

As shown in Figure 2, the tip portion 44 has a tip surface 45 which is provided with an observation optical system 52, illumination optical systems 54, an air-supply/water-supply nozzle 56, and a clamp opening 58. Behind the observation optical system 52 is provided with a solid state image pickup element, which is supported by a substrate that is connected with a signal cable. The signal cable is threaded through the insertion unit 12, the proximal operation unit 14, the universal cable 16, and the like of Figure 1, to be extended to the electrical connector 24 to be connected to a processor 26. The observation optical system 52 captures an observed image, which is focused on a light receiving section of the solid state image pickup element and is converted into an electrical signal. The electrical signal is output to the processor 26 via the signal cable to be converted into a video signal. In this way, the observed image is displayed on a monitor 50 which is connected to the processor 26.

### [First Embodiment]

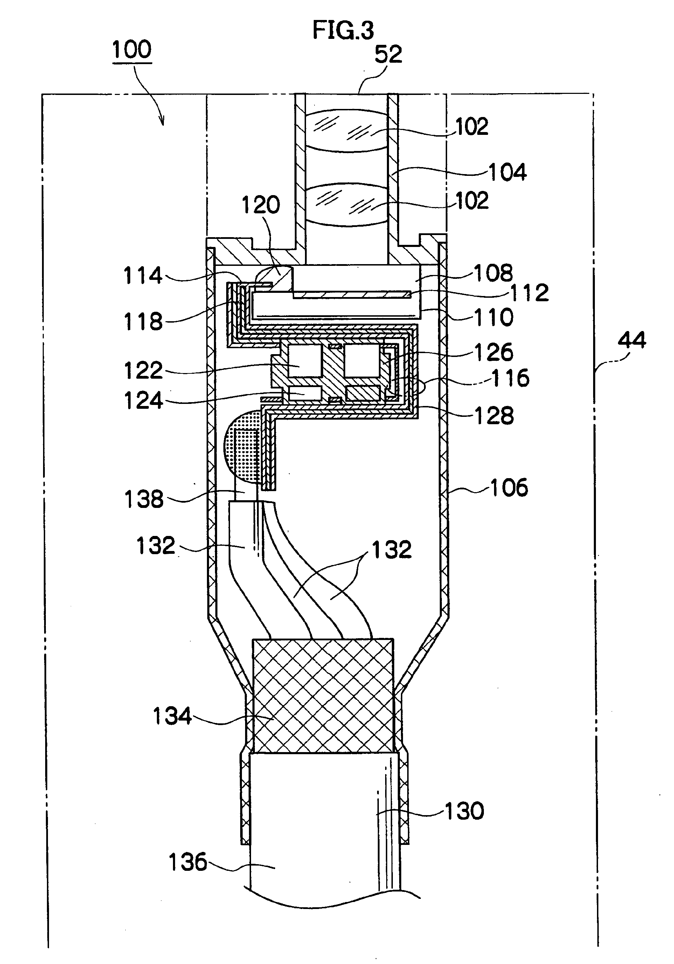

An image pickup apparatus incorporated in the observation optical system will be explained with reference to Figure 3. Figure 3 shows a cross sectional view of an image pickup apparatus 100. The image pickup apparatus 100 includes a lens 102 and a lens barrel 104 for supporting the lens 102. The lens barrel 104 is fixed to a tip portion of a cylinder 106 to tight seal the tip portion of the cylinder 106. The lens barrel 104 and the cylinder 106 forms a space therebetween, in which cover glass 108 is adhesively attached to the lens barrel 104.

The solid state image pickup element 110 is disposed on one side of the lens barrel 104 opposite to the cover glass 108, and is fixed to the cover glass 108. The solid state image pickup element 110 is provided with an electrode 114 at the main surface thereof for input/output signals to and from the light receiving section 112 which converts light signals into electrical signals.

The observed image (light signal) captured by the observation optical system passes through the lens 102 and the cover glass 108 to be focused on the light receiving section 112 of the solid state image pickup element 110, where the light signal is converted into an electrical signal. The electrode 114 is electrically connected to a wiring pattern 118 formed on the flexible circuit board 116. The electrode 114 and the wiring pattern 118 are sealed with a sealing resin 120 in order to increase the connection strength between the electrode 114 and the wiring pattern 118.

The flexible circuit board 116 is manufactured by, for example, sandwiching a conductive member such as copper foil for forming wiring pattern 118 with insulating resin films 128 such as polyimide films. The flexible circuit board 116 can be easily bent due to its small thickness and flexibility.

The flexible circuit board 116 is bent along the solid state image pickup element 110, and is connected to the back surface of the solid state image pickup element 110. The flexible circuit board 116 and the back surface of the solid state image pickup element 110 are connected to each other, by an adhesive (not shown) such as a heat-resistant and electrically insulating resin for example. Thus, the connection does not require a direct contact between the flexible circuit board 116 and the solid state image pickup element 110.

A plurality of electronic components 122, 124 such as an IC, a resistor, a capacitor, and a transistor are mounted only on one surface of the flexible circuit board 116. The flexible circuit board 116 is bent to be connected to the back surface of the solid state image pickup element 110, as a result of that the surface of the flexible circuit board 116 to which the electronic components 122 are mounted is positioned substantially in parallel to the back surface of the solid state image pickup element 110. In the present invention, the surface of the flexible circuit board 116 to which the electronic components 122 are mounted is not bent to be positioned perpendicular to the back surface of the solid state image pickup element 110, thereby the entire length of the image pickup apparatus 100 can be reduced.

The flexible circuit board 116 is bent at a position which does not exceed the projection area of the solid state image pickup element 110, so that the surface of the flexible circuit board 116 to which the electronic components 124 are mounted is disposed substantially in parallel to the back surface of the solid state image pickup element 110. This makes the electronic components 122 and the electronic components 124 disposed opposed to each other in the space which is formed by bending the flexible circuit board 116. Thus, the bending of the flexible circuit board 116 allows the electronic components 122, 124 opposed to each other, which increases the mounting density thereof.

The electronic components 122 and the electronic components 124 are disposed opposed to each other and also in a staggered arrangement without any overlapping, which enables an effective use of the space, and further increases the mounting density thereof.

The electronic components 122 and the electronic components 124 are sealed with a sealing resin 126 in the space. Because the flexible circuit board 116 surrounds the electronic components 122 and the electronic components 124, a filling of the sealing resin 126 into the space easily allows the resin-sealing of the electronic components 122, 124 to be resin sealed. The space formed by the bending of the flexible circuit board 116 serves as a kind of mold for the sealing resin 126.

Then, the flexible circuit board 116 is again bent at another position which does not exceed the projection area of the solid state image pickup element 110, so that the flexible circuit board 116 is electrically connected to wire conductors 138 of a plurality of core wires 132 which are extended from the signal cable 130 by soldering or the like. The core wires 132 supply power for driving the solid state image pickup element 110, and transmit the electrical signal converted at the solid state image pickup element 110 to the processor 26 shown in Figure 1.

The signal cable 130 is configured with the plurality of the core wires 132, an entire braid shield 134 which covers the plurality of the core wires 132 via an insulator (not shown), and an insulating envelope member 136 which covers the entire braid shield 134. The signal cable 130 is fixed to the cylinder 106 at the rear end thereof so as to tight seal the cylinder 106 and the rear end.

The phrase "flexible circuit board 116 is bent at a position which does not exceed the projection area of the solid state image pickup element 110" as used in the present invention means that the flexible circuit board 116 is bent at a position which does not exceed the projection area of the solid state image pickup element 110 between the position where flexible circuit board 116 is in contact with the back surface of the solid state image pickup element 110 and the core wires 132. Therefore, the flexible circuit board 116 may be extended beyond the exterior of the solid state image pickup element 110 between the position where the flexible circuit board 116 is electrically connected with the electrode 114 of the solid state image pickup element 110 and the position where the flexible circuit board 116 is connected to the back surface of the solid state image pickup element 110 after being bent.

In the present invention, in the image pickup apparatus 100, the electronic components 122, 124 are mounted only one surface of the flexible circuit board 116, and the surface of the flexible circuit board 116 to which the electronic components 122, 124 are mounted is disposed substantially in parallel to the back surface of the solid state image pickup element 110, and then the circuit board 116 is bent to be placed within the projection area of the solid state image pickup element 110, which achieves the downsizing of the image pickup apparatus 100 in its longitudinal and radial directions.

In the present invention, the back surface of a solid state image pickup element can be used for mounting, which enhances the reliability in mounting electrical components. In addition, the part for mounting electrical components can be fixed in assembling of an image pickup apparatus, which prevents any disengagement of electrical components in the assembly.

### [Second Embodiment]

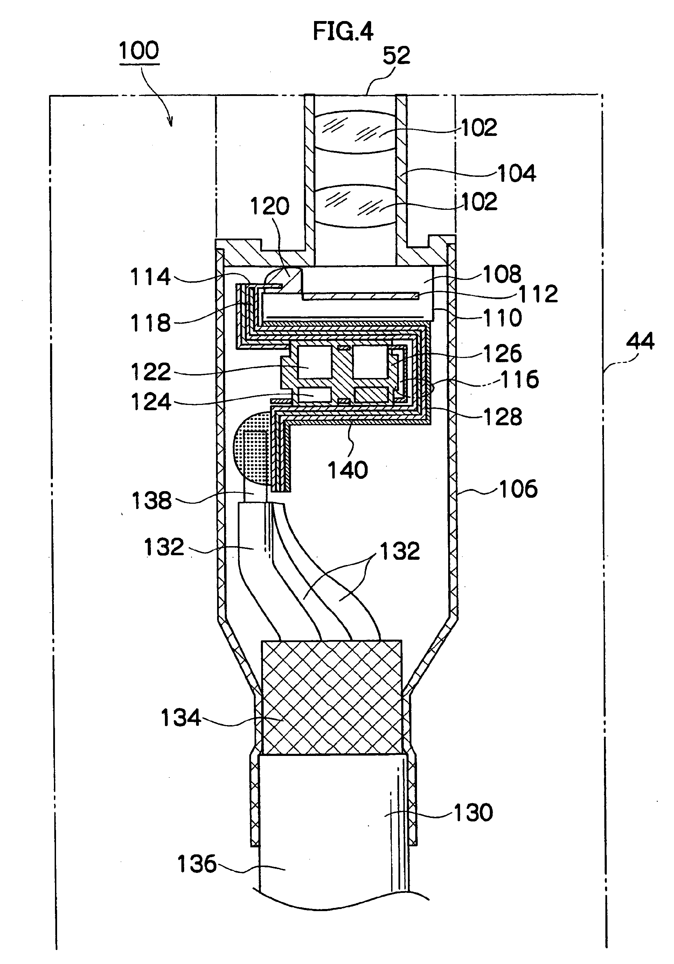

A second embodiment of the present invention will be explained below with reference to Figure 4. Figure 4 shows a cross sectional view of an image pickup apparatus 100. The similar parts of the image pickup apparatus are denoted by the same reference numbers as those in the first embodiment, and may not be explained below.

The image pickup apparatus 100 is configured substantially in the same manner as that in the first embodiment. In the present second embodiment, a flexible circuit board 116 has one surface having electronic components 122, 124 mounted thereto, and the other surface provided with a conductor pattern 140. The conductor pattern 140 is connected to a back surface of a solid state image pickup element 110.

The back surface of the solid state image pickup element 110 is connected to the conductor pattern 140 using an insulating resin having a high thermal conductivity for example, which causes the conductor pattern 140 to function as a radiator plate. The heat generated at the solid state image pickup element 110 is efficiently radiated from the conductor pattern 140 so that any noise of solid state image pickup element 110 caused by the heat can be reduced.

The solid state image pickup element 110 has a main surface to which a cover glass 108 is attached. The cover glass 108 has a hollow structure so that a light receiving section 112 does not contact with the cover glass 108.

The back surface of the solid state image pickup element 110 and the conductor pattern 140 are electrically connected to each other using a conductive adhesive or the like, as a result of that the conductor pattern 140 functions as a GND pattern. The conductor pattern 140 functioning as a GND pattern effects stable driving of the solid state image pickup element 110.

The conductor pattern 140 is formed all over the other surface of the flexible circuit board 116, and surrounds the wiring pattern 118, as a result of that the conductor pattern 140 functions as a shield and reduces the affect by noise from the outside.

A signal cable 130 includes a plurality of core wires 132 having a shield 160. The shield 160 of the core wires 132 is connected to the conductor pattern 140, thereby any electromagnetic waves from the outside can be blocked, and any emission of input/output signals to the outside can be restrained, so as to maintain the quality of the image signals.

Instead of the shield 160 of the core wires 132, the conductor pattern 140 may be connected to an entire braid shield 134 of the signal cable 130. As a result, any electromagnetic waves from the outside can be blocked and any emission of input/output signals to the outside can be restrained, so as to maintain the quality of the image signals.

From the shield of the core wires 132, coatings are removed from the core wires 132 before the connection of the core wires 132, and bundled and connected to the conductor pattern 140 of the flexible circuit board 116 by soldering or the like. From the outermost entire braid shield 134 of the cable 130, the envelope member 136 is peeled off before the connection of the core wires 132 to expose the entire braid shield 134, which is connected to the conductor pattern 140 of the flexible circuit board 116 by soldering or the like.

Alternatively, the conductor pattern 140 may be connected to a cylinder 106. As a result, any electromagnetic waves from the outside can be blocked and any emission of input/output signals to the outside can be restrained, so as to maintain the quality of the image signals. The conductor pattern 140 and the cylinder 106 are electrically and thermally connected to each other by a cable line or a filling of a thermally conductive resin in the cylinder 106.

In addition, the conductor pattern 140 may be connected to a lens barrel 104, so that the heat generated at the solid state image pickup element 110 is transferred to the lens barrel 104 to prevent dew condensation from being formed on the surface of the cover glass 108. A temperature rise due to the heated solid state image pickup element 110 causes a thermal gradient (temperature difference) to be established between the cover glass 108 and the solid state image pickup element 110, which produces dew condensation on the surface of the cover glass 108. When the heat generated at the solid state image pickup element 110 is radiated to the lens barrel 104, the heat generated at the solid state image pickup element 110 is dissipated and also the cover glass 108 is heated, thereby the temperature equilibrium between the cover glass 108 and the solid state image pickup element 110 can be maintained to prevent dew condensation from being formed. The conductor pattern 140 and the lens barrel 104 are connected to each other via a cable line, a conductive resin, or a heat transferring thin film.

### [Third Embodiment]

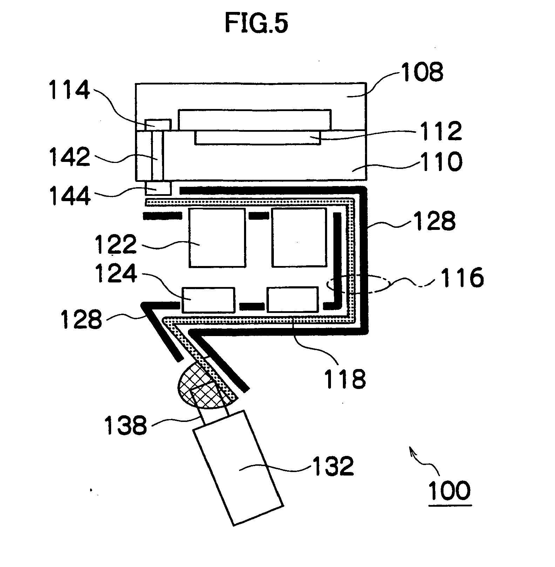

A third embodiment of the present invention will be explained below with reference to Figure 5. Figure 5 partially shows a cross sectional view of an image pickup apparatus 110. The similar parts of the image pickup apparatus are denoted by the same reference numbers as those in the first embodiment, and may not be explained below.

The image pickup apparatus 100 of the third embodiment includes a different connection structure between a flexible circuit board 116 and a solid state image pickup element 110 from those of the first and second embodiments.

A solid state image pickup element 110 is electrically connected to an electrode 114 formed on a main surface thereof, and has a through-hole interconnection I42 formed therein between the main surface and a back surface thereof. The through-hole interconnection 142 is electrically connected to an electrode 144 formed on the back surface of the solid state image pickup element 110. The through-hole interconnection 142 is formed by drilling a through hole below the electrode 114 which is formed on the main surface, and filling a conductive paste into the through hole.

The electrode 144 and a wiring pattern on a flexible circuit board 116 is connected by the conductive paste, ACF (Anisotropic Conductive Film), ultrasonic waves, or the like. However, the connection may be established by other means.

The solid state image pickup element 110 of the present embodiment is manufactured by, for example, a) forming a through-hole interconnection in a process at wafer level, b) attaching a cover glass 108, and c) and cutting using a dicer.

Via the through-hole interconnection 142, the electrode 114 on the main surface and the flexible circuit board 116 are electrically connected to each other, which enables the downsizing of the image pickup apparatus 100 in its radial direction. That is, in a case where the electrical connection between the electrode 114 and the flexible circuit board 116 is provided on the main surface, the flexible circuit board 116 has to be bent along the exterior of the solid state image pickup element 110 to be connected to the back surface of the solid state image pickup element 110. In this case, the flexible circuit board 116 is positioned on the outside of the exterior of the solid state image pickup element 110. To the contrary, in a case where the electrode 114 on the main surface and the flexible circuit board 116 are electrically connected to each other via the through-hole interconnection 142, the flexible circuit board 116 does not have to be bent along the exterior of the solid state image pickup element 110, which enables the downsizing of the image pickup apparatus 100 in its radial direction.

Moreover, since the through-hole interconnection 142 is formed of a conductive paste or the like and has a high thermal conductivity, the heat generated at the solid state image pickup element 110 can be efficiently transferred to the flexible circuit board 116.

### [Method of Assembly]

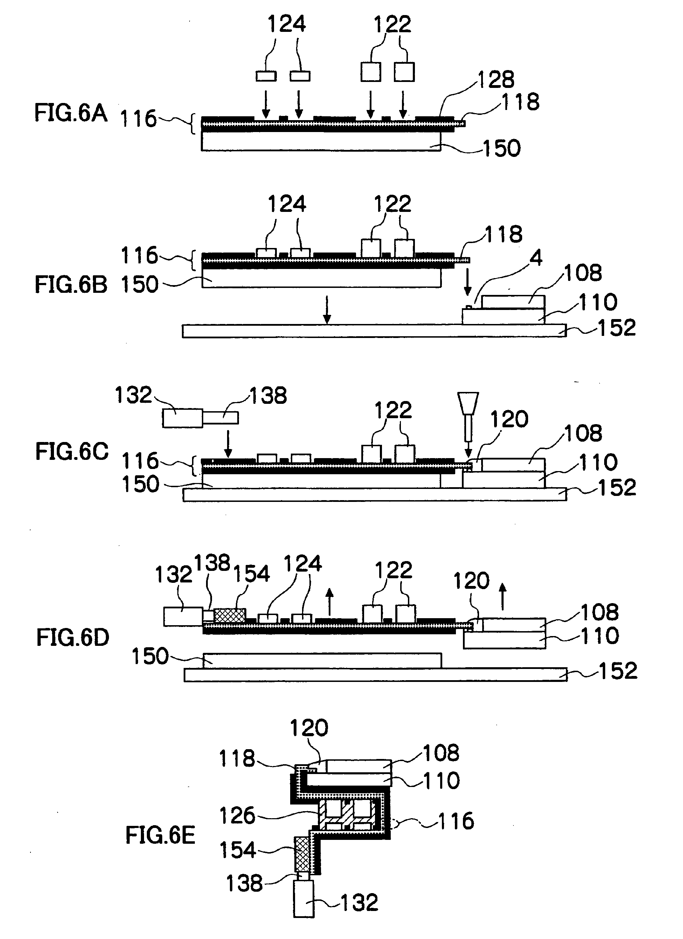

Next, a method for assembling an image pickup apparatus will be explained below with reference to Figures 6A to 6E. As shown in Figure 6A, a flexible circuit board 116 manufactured by sandwiching a wiring pattern 118 formed of copper foil or the like with resin films 128 is provided on a first jig 150, with the surface for mounting of the flexible circuit board 116 facing upward. On the flexible circuit board 116, the resin films 128 on an area where electronic components 122, 124 are mounted, an area for a connection between a solid state image pickup element and an electrode, and an area to be connected with a core wire are removed to expose a wiring pattern 118.

The electronic components 122, 124 are electrically connected only to one surface of the wiring pattern 118 via openings by soldering or the like. Unlike a case of both-side mounting, since the flexible circuit board 116 does not have to be turned around, the electronic components can be easily mounted.

Next, as shown in Figure 6B, a solid state image pickup element 110 having a cover glass 108 attached thereto is prepared on a second jig 152 with a light receiving section thereof facing upward. The wiring pattern 118 is aligned to be over an electrode 114 formed on the solid state image pickup element 110, and then the first jig 150 having the flexible circuit board 116 mounted thereto is placed on the second jig 152.

Next, as shown in Figure 6C, at one end area of the flexible circuit board 116, a wire conductor 138 of core wires 132 and the wiring pattern 118 are electrically connected to each other by soldering 154. At the other end area of the flexible circuit board 116, the wiring pattern 118 and the electrode 114 are electrically connected to each other. In order to secure the connection between the wiring pattern 118 and the electrode 114, the connection is sealed with a resin to form a sealing resin 120.

Next, as shown in Figure 6D, after the electrical connection between the electrode 114, the electronic components 122, 124, and the wire conductor 138 is completed, the flexible circuit board 116 is removed from the first and second jigs 150 and 152.

Then, as shown in Figure 6E, the flexible circuit board 116 is bent to be connected to the back surface of the solid state image pickup element 110, by a heat resistant adhesive for example. The flexible circuit board 116 is bent within the projection area of the solid state image pickup element 110 so that the surface to which the electronic components 122, 124 are mounted is disposed substantially in parallel to the back surface of the solid state image pickup element 110.

The electronic components 122, 124 are received in the space which is formed by the bending of the flexible circuit board 116. A filling of a sealing resin 126 into the space enables the elimination of a frame which has been required in the prior art for surrounding the electronic components.

The surface opposite to the surface to which the electronic components 122, 124 are mounted is fixed to a jig or the like for the mounting and electrical connection as described above, thereby any disengagement of the electrical components due to external forces or vibrations which occur during the manufacturing can be prevented. In addition, the downsized circuit board is fixed to a jig or the like, which assures the handling in the assembling steps.

Moreover, the electrical components are mounted only one surface of a circuit board, which eliminates the necessity of turning around the circuit board and also requires only one reflow or flow step for the mounting, resulting in simplifying the assembling steps.

In the above embodiments, a circuit board has been explained using a flexible circuit board, but is not limited to this, and may be any other circuit board which is flexible, including the one formed of a rigid board such as a glass epoxy board and a flexible board.

## Claims

1. An image pickup apparatus (100), comprising:
a solid state image pickup element (110) which includes a main surface having a light receiving section formed thereon and a back surface;
a bendable circuit board (116) which is electrically connected to the solid state image pickup element via a connection portion;
a signal cable (130) which is electrically connected to the circuit board; and
an electronic component (122) which is mounted to only one surface of the circuit board, wherein
the circuit board (116) is connected to the back surface of the solid state image pickup element (110) and is bent within a projection area of the solid state image pickup element, and
the surface of the circuit board (116) to which the electronic component is mounted is disposed substantially in parallel to the back surface of the solid state image pickup element (110),
**characterized by**
comprising at least two electronic components (122, 124), wherein
the circuit board is bent such that the electronic components are opposed to each other, wherein the opposing electronic components are sealed by resin.

2. The image pickup apparatus according to claim 1, wherein the signal cable is connected to the circuit board at a surface to which the electronic component is mounted.

3. The image pickup apparatus according to claim 1 or 2, wherein the back surface of the solid state image pickup element is connected to the other surface of the circuit board which is opposite to the surface having the electronic component mounted thereto.

4. The image pickup apparatus according to claim 3, wherein the circuit board has a conductor layer on the other surface so that the conductor layer and the back surface of the solid state image pickup element are thermally connected to each other.

5. The image pickup apparatus according to claim 4, wherein the conductor layer is electrically connected to the back surface of the solid state image pickup element.

6. The image pickup apparatus according to claim 4 or 5, wherein the conductor layer is connected to an entire shield of the signal cable.

7. The image pickup apparatus according to any one of claims 4 to 6, further comprising a cylinder which partly covers the solid state image pickup element, the circuit board, the electronic component, and the signal cable, wherein
the cylinder and the conductor layer are connected to each other.

8. The image pickup apparatus according to any one of claims 4 to 7, further comprising a lens barrel which is provided on the main surface of the solid state image pickup element for holding an optical system,
wherein
the lens barrel and the conductor are connected to each other.

9. The image pickup apparatus according to claim 1, wherein the opposing electronic components are in a staggered arrangement.

10. The image pickup apparatus according to any one of claims 1 to 9, wherein the connection is a through-hole interconnection formed in the solid state image pickup element.

11. The image pickup apparatus according to claim 10, further comprising an underfill agent which is filled between the solid state image pickup element and the circuit board.

12. The image pickup apparatus according to any one of claims 1 to 9, wherein the circuit board (116) and the solid state image pickup element (110) are electrically connected to each other on the main surface of the solid state image pickup element (110).

13. An endoscope comprising the image pickup apparatus according to any one of claims 1 to 12.

## Patentansprüche

1. Bildaufnahmegerät (100), umfassend:
ein Festkörper-Bildaufnahmeelement (110), welches eine Hauptfläche mit einem darauf ausgebildeten Lichtaufnahmeabschnitt und eine Rückseite aufweist;
eine biegbare Schaltungsplatine (116), die elektrisch über einen Verbindungsteil mit dem Festkörper-Bildaufnahmeelement verbunden ist;
ein Einzelkabel (130), welches elektrisch mit der Schaltungsplatine verbunden ist; und
eine Elektronikkomponente (122), die auf lediglich einer Fläche der Schaltungsplatine angebracht ist, wobei
die Schaltungsplatine (116) mit der Rückseite des Festkörper-Bildaufnahmeelements (110) verbunden ist und innerhalb eines Projektionsbereichs des Festkörper-Bildaufnahmeelements gebogen ist, und
die Oberfläche der Schaltungsplatine (116), an der die Elektronikkomponente angebracht ist, sich im wesentlichen parallel zu der Rückseite des Festkörper-Bildaufnahmeelements (110) befindet,
**gekennzeichnet durch**
mindestens zwei Elektronikkomponenten (122, 124), wobei die Schaltungsplatine derart gebogen ist, dass die Elektronikkomponenten einander gegenüberliegen, wobei die einander gegenüberliegenden Elektronikkomponenten **durch** Harzmaterial vergossen sind.

2. Bildaufnahmegerät nach Anspruch 1, bei dem das Signalkabel mit der Schaltungsplatine an einer Fläche verbunden ist, an der die Elektronikkomponente angebracht ist.

3. Bildaufnahmegerät nach Anspruch 1 oder 2, bei dem die Rückseite des Festkörper-Bildaufnahmeelements mit der anderen Fläche der Schaltungsplatine verbunden ist, die der Fläche abgewandt ist, an der die Elektronikkomponente angebracht ist.

4. Bildaufnahmegerät nach Anspruch 3, bei der die Schaltungsplatine eine Leiterschicht auf der anderen Fläche ist, so dass die Leiterschicht und die Rückseite des Festkörper-Bildaufnahmeelements thermisch miteinander verbunden sind.

5. Bildaufnahmegerät nach Anspruch 4, bei dem die Leiterschicht elektrisch mit der Rückseite des Festkörper-Bildaufnahmeelements verbunden ist.

6. Bildaufnahmegerät nach Anspruch 4 oder 5, bei dem die Leiterschicht mit einer vollständigen Abschirmung des Signalkabels verbunden ist.

7. Bildaufnahmegerät nach einem der Ansprüche 4 bis 6, weiterhin umfassend einen Zylinder, der das Festkörper-Bildaufnahmeelement, die Schaltungsplatine, die Elektronikkomponente und das Signalkabel teilweise bedeckt, wobei
der Zylinder und die Leiterschicht miteinander verbunden sind.

8. Bildaufnahmegerät nach einem der Ansprüche 4 bis 7, weiterhin umfassend einen Objektivtubus, der an der Hauptfläche des Festkörper-Bildaufnahmeelements zum Halten einer Optik vorgesehen ist, wobei
der Linsentubus und der Leiter miteinander verbunden sind.

9. Bildaufnahmegerät nach Anspruch 1, bei dem die einander gegenüberliegende Elektronikkomponenten versetzt angeordnet sind.

10. Bildaufnahmegerät nach einem der Ansprüche 1 bis 9, bei dem die Verbindung eine Durchkontaktierungslochverbindung ist, die in dem Festkörper-Bildaufnahmeelement ausgebildet ist.

11. Bildaufnahmegerät nach Anspruch 10, weiterhin umfassend ein Unterfüllungsmittel, welches zwischen das Festkörper-Bildaufnahmeelement und die Schaltungsplatine gefüllt ist.

12. Bildaufnahmegerät nach einem der Ansprüche 1 bis 9, bei dem die Schaltungsplatine (116) und das Festkörper-Bildaufnahmeelement (110) elektrisch miteinander auf der Hauptfläche des Festkörper-Bildaufnahmeelements (110) verbunden sind.

13. Endoskop, umfassend ein Bildaufnahmegerät nach einem der Ansprüche 1 bis 12.

## Revendications

1. Appareil de prise de vues (100), comprenant :
un élément de prise de vues à semi-conducteurs (110) qui comporte une surface principale possédant une section réceptrice de lumière formée sur celui-ci et une surface arrière ;
une carte de circuit imprimé pliable (116) qui est connectée électriquement à l'élément de prise de vues à semi-conducteurs par le biais d'une partie de connexion ;
un câble d'interface (130) qui est connecté électriquement à la carte de circuit imprimé ; et
un composant électronique (122) qui est monté sur une seule surface de la carte de circuit imprimé, dans lequel
la carte de circuit imprimé (116) est connectée à la surface arrière de l'élément de prise de vues à semi-conducteurs (110) et est repliée à l'intérieur d'une zone de projection de l'élément de prise de vues à semi-conducteurs, et
la surface de la carte de circuit imprimé (116) sur laquelle est monté le composant électronique est disposée sensiblement en parallèle à la surface arrière de l'élément de prise de vues à semi-conducteurs (110),
**caractérisé en ce que**
il comprend au moins deux composants électroniques (122, 124),
la carte de circuit imprimé étant repliée de telle sorte que les composants électroniques sont opposés l'un à l'autre, les composants électroniques opposés étant scellés par de la résine.

2. Appareil de prise de vues selon la revendication 1, dans lequel le câble d'interface est connecté à la carte de circuit imprimé au niveau d'une surface sur laquelle est monté le composant électronique.

3. Appareil de prise de vues selon la revendication 1 ou 2, dans lequel la surface arrière de l'élément de prise de vues à semi-conducteurs est connectée à l'autre surface de la carte de circuit imprimé qui est opposée à la surface sur laquelle est monté le composant électronique.

4. Appareil de prise de vues selon la revendication 3, dans lequel la carte de circuit imprimé possède une couche conductrice sur l'autre surface de telle sorte que la couche conductrice et la surface arrière de l'élément de prise de vues à semi-conducteurs sont thermiquement connectées l'une à l'autre.

5. Appareil de prise de vues selon la revendication 4, dans lequel la couche conductrice est électriquement connectée à la surface arrière de l'élément de prise de vues à semi-conducteurs.

6. Appareil de prise de vues selon la revendication 4 ou 5, dans lequel la couche conductrice est connectée à un blindage entier du câble d'interface.

7. Appareil de prise de vues selon l'une quelconque des revendications 4 à 6, comprenant en outre un cylindre qui couvre partiellement l'élément de prise de vues à semi-conducteurs, la carte de circuit imprimé, le composant électronique et le câble d'interface, dans lequel
le cylindre et la couche conductrice sont connectés l'un à l'autre.

8. Appareil de prise de vues selon l'une quelconque des revendications 4 à 7, comprenant en outre un barillet d'objectif qui est placé sur la surface principale de l'élément de prise de vues à semi-conducteurs pour maintenir un système optique, dans lequel
le barillet d'objectif et le conducteur sont connectés l'un à l'autre.

9. Appareil de prise de vues selon la revendication 1, dans lequel les composants électroniques opposés sont disposés en quinconce.

10. Appareil de prise de vues selon l'une quelconque des revendications 1 à 9, dans lequel la connexion est une interconnexion par trou traversant formée dans l'élément de prise de vues à semi-conducteurs.

11. Appareil de prise de vues selon la revendication 10, comprenant en outre un agent de sous-remplissage qui remplit l'espace entre l'élément de prise de vues à semi-conducteurs et la carte de circuit imprimé.

12. Appareil de prise de vues selon l'une quelconque des revendications 1 à 9, dans lequel la carte de circuit imprimé (116) et l'élément de prise de vues à semi-conducteurs (110) sont connectés électriquement l'un à l'autre sur la surface principale de l'élément de prise de vues à semi-conducteurs (110).

13. Endoscope comprenant l'appareil de prise de vues selon l'une quelconque des revendications 1 à 12.
